# EUROPEAN PATENT APPLICATION

(11) **EP 4 353 302 A1**
(43) Date of publication of application: **17.04.2024**
(21) Application number: 23202784.7
(22) Date of filing: 10.10.2023
(51) Int. Cl.: A61N 1/36, A61N 1/375, A61N 1/378

(54) **IMPLANTABLE STIMULATOR WITH EXTERNALIZED BATTERY**

(30) Priority: 12.10.2022 US 202263379261 P; 20.09.2023 US 202318470721
(71) Applicant: Medtronic, Inc., Minneapolis, MN 55432 (US)
(72) Inventor: Iyer, Rajesh V., Brooklyn Center (US); Thom, Andrew J., Brooklyn Center (US); Young, Paul B., Brooklyn Center (US); Jain, Gaurav, Brooklyn Center (US); Gaddam, Venkat R., Minneapolis (US); Mehra, Ashutosh, Minneapolis (US); Schmidt, Craig L., Brooklyn Center (US)
(74) Representative: Zimmermann & Partner Patentanwälte mbB

(57) **Abstract**

An example medical device includes a device housing configured to be implantable within a patient, the device housing including an internal surface in contact with a voltaic cell of the battery, and a battery external to the device housing and comprising a battery housing configured to be hermetically sealed. The battery is configured to provide electrical power to an electrical component housed within the device housing, and the battery housing is configured to be attached to the device housing. The battery housing includes an internal surface in contact with a voltaic cell of the battery, and an external surface in contact with the biocompatible electrical insulator.an external surface in contact with the biocompatible electrical insulator.

## Description

This application claims the benefit of U.S. Provisional Patent Application 63/379,261, filed on October 12, 2022.

### TECHNICAL FIELD

The disclosure relates to medical devices and, more particularly, medical devices that deliver therapy to a patient.

### BACKGROUND

Implantable medical devices are configured to deliver electrical stimulation therapy or monitor physiological signals. Electrical stimulation of nerve tissue, for example, may provide relief for a variety of disorders thereby improving the quality of life for many patients.

Disease, age, and injury may impair physiological functions of a patient. In some situations, the physiological functions are completely impaired. In other examples, the physiological function may operate sufficiently at some times or under some conditions and operate inadequately at other times or at other conditions. In one example, bladder dysfunction, such as overactive bladder, urgency, or urinary incontinence, is a problem that may afflict people of all ages, genders, and races. Various muscles, nerves, organs and conduits within the pelvic floor cooperate to collect, store and release urine. A variety of disorders may compromise urinary tract performance, and contribute to an overactive bladder, urgency, or urinary incontinence that interferes with normal physiological function. Many of the disorders may be associated with aging, injury or illness.

Urinary incontinence may include urge incontinence and stress incontinence. In some examples, urge incontinence may be caused by disorders of peripheral or central nervous systems that control bladder micturition reflexes. Some patients may also suffer from nerve disorders that prevent proper triggering and operation of the bladder, sphincter muscles or nerve disorders that lead to overactive bladder activities or urge incontinence. In some cases, urinary incontinence may be attributed to improper sphincter function, either in the internal urinary sphincter or external urinary sphincter.

### SUMMARY

In general, the disclosure is directed to devices, systems, and techniques for improving battery capacity for power delivery to electronics in an implantable medical device (IMD), such as an implantable stimulation device. In some examples, this disclosure is directed to devices, systems, and techniques for increasing the battery capacity of an IMD without increasing the overall size and/or volume of the IMD. An example medical device includes a device housing configured to be implantable within a patient, the device housing including an internal surface in contact with a voltaic cell of the battery, and a battery external to the device housing and comprising a battery housing configured to be hermetically sealed. The battery is configured to provide electrical power to an electrical component housed within the device housing, and the battery housing is configured to be attached to the device housing. The battery housing includes an internal surface in contact with a voltaic cell of the battery, and an external surface in contact with the biocompatible electrical insulator.an external surface in contact with the biocompatible electrical insulator.

Implantable medical devices, such as tibial stimulators, neurostimulators, neuromonitoring devices, or the like, may benefit from increased battery capacity without an increase in the volume and/or surface area of the IMD. To increase battery capacity, the size of a battery may be increased at the cost of decreasing and/or reducing non-battery components, e.g., in order to avoid increasing the overall size/volume of the IMD. In examples described herein, battery size may be increased by eliminating a portion of the IMD housing by "externalizing" the battery. For example, rather than enclosing a battery within the housing of an IMD, the battery may be externalized by attaching the battery to an external portion of the IMD housing. An externalized battery may be referred to as a "single hull" battery because the battery portion of a device (e.g., an IMD) utilizing the battery includes a single housing and/or enclosure for the battery cells, voltaic cells, battery materials, and/or battery chemicals. A battery that is not externalized is a battery which is housed within the device housing. Such a battery will have its own battery housing or enclosure, and is additionally housed within an enclosure of the device. Such a non-externalized battery may be referred to has a "double hull" battery because the battery portion of a device (e.g., an IMD) utilizing the battery includes a two housings and/or enclosures, e.g., a device housing that houses the battery and a battery housing that houses the battery cells, voltaic cells, battery materials, and/or battery chemicals. A separate, double-hulled battery may be "external" to a device and have a double hull, e.g., a battery having its own battery housing that is also housed within a housing configured to attach to a device housing. In examples described herein, an externalized battery may be a single hull including a battery housing that both houses the battery cells, voltaic cells, battery materials, and/or battery chemistry, and is configured to be attached to an external portion of the device utilizing the battery, e.g., an IMD.

In one or more examples, an externalized battery is both external to the IMD housing and does not include a separate housing. The battery housing of an externalized battery that is configured to enclose the battery cells, voltaic cells, battery materials, and/or battery chemicals is exposed to the same environment as the IMD housing, e.g., air, bodily tissue and/or fluids (when implanted), a device coating, or the like. The battery housing of an externalized battery may comprise, or be connected to, a terminal of the battery, and as such, a terminal of example externalized batteries disclosed herein may include a battery terminal having a surface exposed to the same environment as an external surface of the IMD housing, e.g., air, bodily tissue and/or fluids, a device coating, or the like, because example externalized batteries disclosed herein may comprise single-hulled batteries.

An externalized battery is distinguishable from a separate, double-hulled battery, where the separate, double-hulled battery may be attached to an IMD but not within the housing of the IMD. As discussed above, an separate, double-hulled battery is encased in a separate housing that is separate from the IMD housing and is configured to mate with or be attached to the IMD housing. However, the battery still includes a battery housing enclosing the battery chemistry within the separate housing, e.g., it is double-hulled. A separate, double-hulled battery does not have a battery terminal having a surface exposed to the same environment as an external surface of the IMD housing, e.g., air, bodily tissue and/or fluids, a device coating, or the like, because the battery housing of the separate, double-hulled battery that is configured to enclose battery cells, voltaic cells, battery materials, and/or battery chemicals is housed within the separate housing.

Accordingly, the techniques may provide one or more technical advantages that realize at least one practical application. For example, the systems, devices, and techniques disclosed may improve battery capacity and longevity of an IMD without increasing the overall size, volume, and/or surface area of the IMD. In some examples, the systems, devices, and techniques may improve the recharge of the IMD, and may provide improved wireless communications, e.g., via at least a portion of the IMD housing and/or battery housing comprising radio frequency (RF) transparent and/or non-shielding materials.

In one example, this disclosure describes a medical device including: a device housing configured to be implantable within a patient, the device housing including an external surface in contact with a biocompatible electrical insulator; an electrical component housed within the device housing; and a battery external to the device housing and comprising a battery housing configured to be hermetically sealed, wherein the battery is configured to provide electrical power to the electrical component housed within the device housing, wherein the battery housing is configured to be attached to the device housing, wherein the battery housing includes: an internal surface in contact with a voltaic cell of the battery; and an external surface in contact with the biocompatible electrical insulator.an external surface in contact with the biocompatible electrical insulator.

In another example, this disclosure describes a medical device including: stimulation circuitry configured to generate an electrical stimulation signal; and a device housing configured to be implantable within a patient; wherein the device housing includes: a first portion housing the stimulation circuitry within a first volume; and a second portion housing a battery within a second volume, wherein the second portion forms a hermetic seal of the second volume, and wherein the battery is configured to provide electrical power to the stimulation circuitry.

In another example, this disclosure describes a method of manufacturing a medical device, the method including: hermetically sealing a voltaic cell within a battery housing configured to be implantable within a patient; and externally attaching the battery housing to a device housing configured to be implantable within a patient, wherein the battery housing includes: an internal surface in contact with a voltaic cell of the battery; and an external surface exposed to substantially the same environment as an external surface of the device housing.

The details of one or more examples are set forth in the accompanying drawings and the description below. Other features, objects, and advantages of the disclosure will be apparent from the description and drawings, and from the claims.

The above summary is not intended to describe each illustrated example or every implementation of the present disclosure.

### BRIEF DESCRIPTION OF DRAWINGS

FIG. 1 is a schematic diagram illustrating an example medical device.
FIG. 2 is a schematic diagram illustrating an example medical device.
FIG. 3 is a schematic diagram illustrating an example medical device.
FIG. 4 is a schematic diagram illustrating an example medical device.
FIG. 5 is a schematic diagram illustrating an example medical device.
FIG. 6 is a conceptual diagram illustrating a leg having a medical device implanted therein near a tibial nerve.
FIG. 7 is a conceptual diagram illustrating an example system that manages delivery of neurostimulation to a patient to manage bladder dysfunction, such as overactive bladder, urgency, or urinary incontinence.
FIG. 8 is a block diagram illustrating an example configuration of an implantable medical devices (IMD).
FIG. 9 is a block diagram illustrating an example configuration of an external programmer.
FIG. 10 is a flow diagram illustrating an example method of manufacturing a medical device.

### DETAILED DESCRIPTION

The disclosure is directed to devices, systems, and techniques for improving battery capacity for power delivery to electronics in an implantable medical device (IMD), such as an implantable stimulation device, without increasing the overall size, volume, and/or surface area of the IMD. These techniques may be used with a neurostimulator that may provide therapy for a variety of dysfunctions, diseases or disorders. For purposes of illustration, but without limitation, use of the techniques of this disclosure will be described below with respect to bladder dysfunction. Bladder dysfunction generally refers to a condition of improper functioning of the bladder or urinary tract, and may include, for example, an overactive bladder, urgency, or urinary incontinence. Overactive bladder (OAB) is a patient condition that may include symptoms, such as urgency, with or without urinary incontinence. Urgency is a sudden, compelling urge to urinate, and may often, though not always, be associated with urinary incontinence. Urinary incontinence refers to a condition of involuntary loss of urine, and may include urge incontinence, stress incontinence, or both stress and urge incontinence, which may be referred to as mixed urinary incontinence. As used in this disclosure, the term "urinary incontinence" includes disorders in which urination occurs when not desired, such as stress or urge incontinence. Other bladder dysfunctions may include disorders such as non-obstructive urinary retention.

One type of therapy for treating bladder dysfunction includes delivery of continuous or periodic electrical stimulation to a target tissue site within a patient to cause a therapeutic effect during delivery of the electrical stimulation. For example, delivery of electrical stimulation from an implantable medical device (IMD) to a target therapy site, e.g., a tissue site that delivers stimulation to modulate activity of a tibial nerve, spinal nerve (e.g., a sacral nerve), a pudendal nerve, dorsal genital nerve, an inferior rectal nerve, a perineal nerve, or branches of any of the aforementioned nerves, may provide an immediate therapeutic effect for bladder dysfunction, such as a desired reduction in frequency of bladder contractions. In some cases, electrical stimulation of the tibial nerve may modulate afferent nerve activities to restore urinary function during the electrical stimulation. Delivery of electrical stimulation (which may include pulsed stimulation) or other types of neurostimulation (e.g., drug delivery therapy) uses energy and depletes the battery of the IMD. The longevity of the battery, and the functioning of the IMD, may be increased by using a larger battery, at the cost of increasing the size and weight of the IMD that is implanted in a patient.

The systems, devices, and techniques described herein provide an IMD with an increased capacity without increasing the overall size, volume, and/or surface area of the IMD. In some examples, an IMD battery (i.e., battery for the IMD) is an externalized battery that is external to the housing of the IMD and includes only a housing of the battery cells and/or chemistry, e.g., a single-hull battery. In some examples, an externalized battery may be configured to hermetically seal the battery cells and/or battery chemistry, and the externalized battery may be configured to provide electrical power to electrical components housed within the IMD housing, e.g., an electrode, circuitry, or the like. For example, the housing of the externalized battery may enclose a volume including a voltaic cell, as an example, and may include an internal surface in contact with a voltaic cell and/or battery chemistry and/or materials, and may include an external surface exposed to the same environment as the external surface of the IMD housing, e.g., air, bodily tissues and/or fluids, and/or a coating on the external surface of the IMD and battery. In some examples, the externalized battery provides a larger battery by way of elimination of a portion of the IMD housing, e.g., a portion of the IMD housing is replaced with a larger battery configured to be exposed to the same environment as the housing of the IMD. In some examples, the externalized battery may be a primary battery (e.g., comprising one or more primary cell) or a rechargeable battery (e.g., comprising one or more rechargeable cell). In some examples, the externalized battery may be a lithium-ion battery.

The disclosure includes discussion of various examples, aspects, and features. Unless otherwise stated, the various examples, aspects, and features are contemplated as being used together in different combinations. For ease of discussion and as a practical matter, each possible combination of features is not expressly recited.

In some examples, a system may be configured to provide stimulation at a neural target located at a distant site from the end organ that is being affected. For example, stimulation sites may be located a relatively large distance from the bladder or bowel, such as a tibial nerve. The system may include multiple devices (e.g., implantable sensors and implantable stimulation devices) with wireless communication circuitry that allows for wireless communication of information between the devices, which may provide sensing or therapeutic stimulation. For example, the wireless circuitry may be designed to communicate using Near Field Communication, Bluetooth^{®}, or other wireless protocols.

For tibial stimulation, the techniques of this disclosure may increase the capacity of the battery by at least 1%, or by at least 10%, or by at least 50%, or by at least 100%. In some examples, a clinician may recharge the externalized or extendable battery of the IMD during an office visit. In such a case, the IMD may appear to be similar to a primary cell device to the patient.

For ease of discussion, various examples are discussed in connection with bladder function. It is to be recognized that bladder function is but one possible application. Various aspects of the present disclosure may also be used in connection with urinary, bowel, and general pelvic floor dysfunction. For the sake of brevity, each type of dysfunction is not repeated for each feature or example discussed herein.

Constant or continuous stimulation may result in undesirable side effects, accommodation, less focused therapy, and increased energy usage by the medical device delivering therapy. For instance, for tibial stimulation, the IMD may provide stimulation for 30 minutes once a week, and the patient may experience relief.

Therefore, stimulation may be cycled on and off. When stimulation is cycled off, the neurostimulation device may enter a hibernation mode in which very little current is drawn from the battery powering the neurostimulation device. This may translate into longer recharge intervals and/or longer replacement intervals.

A medical device, such as an IMD, may implement the techniques described in this disclosure to deliver stimulation therapy to at least one nerve (e.g., tibial nerve, a spinal nerve, or a pelvic floor nerve) to modulate activity of the nerve via at least one electrode electrically connected to the IMD. The electrical stimulation may be configured to modulate contraction of a detrusor muscle of the patient to cause a decrease in frequency of bladder contractions (to reduce incontinence) or an increase in the frequency of bladder contractions (to promote voiding). Reduction in frequency of bladder contractions may reduce urgency of voiding and may reduce urgency and/or urinary incontinence, and thereby at least partially alleviate bladder dysfunction. As another example, electrical stimulation, such as to a tibial nerve, may cause the brain of the patient to ignore signals requesting to void, thereby reducing the chances that the patient voids at undesirable times.

The neurostimulation described herein may be targeted to manage bladder dysfunction, such as an overactive bladder, urgency, urinary incontinence, or even non-obstructive urinary retention. For example, the stimulation may be delivered to target tissue sites normally used to alleviate these types of dysfunction. Although the techniques are primarily described in this disclosure for managing bladder dysfunction, the techniques may also be applied to manage other pelvic floor disorders or disorders relating to other organs, tissues or nerves of the patient. For example, the devices, systems, and techniques described in this disclosure alternatively or additionally may be utilized to manage sexual dysfunction, pelvic pain, fecal urgency or fecal incontinence. Example nerves that may be targeted for therapy include tibial nerves, sacral nerves, pudendal nerves, a dorsal nerve of the penis or clitoris, sural nerves, sciatic nerves, the inferior rectal nerve, and peroneal or perineal nerves. Example organ systems that may be treated for dysfunction may include the large and small bowel, stomach and/or intestines, liver, and spleen, which may be modulated by delivering neurostimulation directly to the organs, to one or nerves innervating the organ, and/or blood supplies reaching the organs. In other examples, therapy may target a spinal cord for pain relief. In other examples, therapy may target a brain for treatment of Parkinson's disease or seizure disorders.

Various examples are discussed relative to one or more stimulation devices. It is recognized that the stimulation devices may include features and functionality in addition to electrical stimulation. Many of these additional features are expressly discussed herein. A few example features include, but are not limited to, different types of sensing capabilities and different types of wireless communication capabilities. For ease of discussion, the present disclosure does not expressly recite every conceivable combination the additional features, such as by repeating every feature each time different examples and uses of the stimulation devices are discussed.

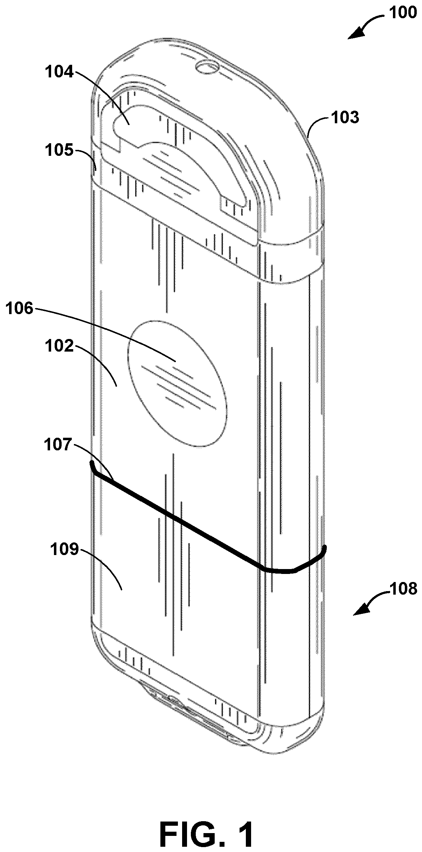

FIG. 1 is a schematic diagram illustrating an example medical device 100, also referred to as IMD 100. IMD 100 includes a device housing 102 containing one or more electrical components therein, such as circuitry, antennas, electrode connections, or the like. In some examples, IMD 100 may be a leadless neurostimulation device configured for delivering neurostimulation therapy. In the example shown, IMD 100 includes a header unit 103 that includes one or more primary electrodes 104, and a mounting plate 105 that couples device housing 102 to header unit 103. Header unit 103 includes at least one primary electrode 104 that forms part of an exterior surface of header unit 103. Device housing 102 includes a secondary electrode 106 that forms part of an exterior surface of device housing 102 and is positioned on the same side of IMD 100 as primary electrode 104. In another example, primary electrode 104 and secondary electrode 106 may be arranged on opposite sides of IMD 100.

Primary electrode 104 and secondary electrode 106 operate in conjunction with one another to provide stimulation therapy to a target treatment site (e.g., a tibial nerve). Secondary electrode 106 may also be referred to as a case electrode, can electrode or reference electrode. In an example, primary electrode 104 may comprise a cathode and secondary electrode 106 may comprise an anode. As described in more detail, in some examples, primary electrode 104 may comprise an anode, and secondary electrode 106 may comprise a cathode. In some examples, primary and secondary electrodes 104 and 106 may be characterized as a bipolar pair or system.

The terms "primary" and "secondary" are used to differentiate two or more electrodes that are configured to transmit an electrical signal therebetween. The terms are not used to imply a hierarchy among the electrodes, positive and negative terminal, a total number of electrodes, or a directionality by which a signal is transmitted between the electrodes. Additional information relating to medical device 100 may be found in U.S. Patent Publication 2022/0096845A1, the entirety of which is incorporated herein by reference.

In the example shown, IMD 100 also includes externalized battery 108. Externalized battery 108 comprises battery housing 109 that is external to device housing 102 and may be attached to device housing 102, e.g., via attachment 107. In some examples device housing 102 and battery housing 109 may comprise a biocompatible material, such as titanium, a ceramic such as a single-crystal aluminum oxide (e.g., sapphire), a polycrystalline alumina, or the like. For example, device housing 102 and battery housing 109 are configured to be implantable within a patient. In some examples, one or both of device housing 102 and battery housing 109 may be configured to form a hermetic seal to enclose a volume and/or configured to be joined to metal, e.g., via attachment 107. In some examples, attachment 107 may comprise an adhesive, a mechanical mechanism configured to hold battery housing 109 to be proximate and/or adjacent to device housing 102, a weld, e.g., a titanium weld ring or ferrule that is brazed or comprises a diffusion bond, or the like.

In some examples, battery 108 is configured to be modular, e.g., changeable or swappable without changing coupling connections and/or circuitry of IMD 100. For example, battery 108 may be detached at attachment 107 and removed from IMD 100. A separate externalized battery, e.g., a new or different externalized battery that may be smaller, the same size, or larger than externalized battery 108, may then be attached to device housing 102 via attachment 107 (e.g., adhered, mechanically coupled, welded, bonded, or the like). If such a separate externalized battery is smaller or larger than externalized battery 108 but otherwise substantially similar to externalized battery 108, the overall size of IMD 100 may change, however, the battery capacity of the separate externalized battery per IMD 100 volume is greater than an alternative separate and/or double-hulled battery.

In some examples, IMD 100 includes a biocompatible electrical insulator (not shown). For example, the external surfaces of device housing 102 and battery housing 109 may be configured to be in contact with, e.g., coated with parylene, with the exception of primary electrode 104 and secondary electrode 106. The parylene coating may be configured to electrically isolate device housing 102 and battery housing 109 from tissue and/or fluids of the patient, and/or increase an electrical resistance between device housing 102 (and battery housing 109) and tissue and/or fluids of the patient (with the exception of the surface area of primary electrode 104 and secondary electrode 106).

In the example shown, externalized battery 108 is external to device housing 102, and battery housing 109 is configured to be hermetically sealed. For example, battery housing 109 is configured to hermetically seal one or more battery cells, voltaic cells, battery chemistry and/or materials, or the like, and to provide one or more electrical connections or contact points to device housing 102 and/or one or more electrical components housed within device housing 102. In other words, externalized battery 108 is configured to provide electrical power to an electrical component housed within device housing 102.

In some examples, battery housing 109 comprises a metal, such as titanium, or a ceramic, such as sapphire. Battery housing 109 comprises an internal surface configured to be in contact with a volume, hermetically sealed by battery housing 109, containing one or more battery cells, one or more voltaic cells, and/or battery materials or battery chemistry. In some examples, the inner surface of battery housing 109 may be in contact with the one or more battery cells, the one or more voltaic cells, and/or the battery materials or battery chemistry. In some examples, battery housing 109 comprises an external surface configured to be in contact with the same environment as the external surface of device housing 102. For example, the external surface of battery housing 109 may be in contact with bodily tissues and/or fluids, or a biocompatible electrical insulator, e.g., the external surface of battery housing 109 may be configured to be coated with parylene.

The internal surface and external surface of battery housing 109 are opposing surfaces, e.g., of the same battery housing wall. In other words, IMD 100 may comprise a single-hulled externalized battery 108 external to device housing 102 and configured to increase a battery capacity of IMD 100 without increasing the overall size, volume, and/or surface area of IMD 100 relative to an IMD including a battery within the IMD housing (e.g., within device housing 102 if device housing 102 were to be extended to include the surface area and/or volume of battery housing 109).

In some examples, battery housing 109 may comprise a conductive material (e.g., titanium) and is configured to be separate from the positive and negative terminals of battery 108, e.g., battery housing 109 is at a neutral or floating electrical potential and may be referred to as "case neutral." In other examples, battery housing 109 is configured to be connected to, or to comprise, a terminal of the battery. For example, battery housing 109 may be configured to be, or be electrically connected to, the positive terminal of battery 108 and may be "case positive," e.g., battery housing may be at a positive electrical potential. In examples in which battery housing 109 is case neutral or case positive, IMD 100 may be configured to electrically isolate battery housing 109 from device housing 102 and primary and secondary electrodes 104, 106. In other examples, battery housing 109 may be configured to be, or be electrically connected to, the negative terminal of battery 108 and may be "case negative," e.g., battery housing may be at a negative electrical potential.

In some examples, battery housing 109 may be in electrical contact with device housing 102. For example, battery housing 109 may have a low electrical resistance path between device housing 102 and battery housing 109, e.g., battery housing 109 may be shorted or "shunted" to device housing 102. In such examples, battery housing 109 may be, or may be connected to, a negative terminal of battery 108 and be case negative. For example, if battery housing 109 is in electrical contact with housing 102, there may be a direct electrical current path from secondary electrode 106 to battery housing 109, e.g., battery housing 109 may be shunted to secondary electrode 106 via device housing 102. If battery housing 109 is not in direct electrical contact with device housing 102 such that there is no direct, e.g., relatively low electrical resistance, path between device housing 102 and battery housing 109, battery housing 109 may still be shunted to secondary electrode 106 via tissue and/or fluids of the patient (e.g., by virtue of being proximate or relatively near device housing 102). If battery housing 109 is case positive, IMD 100 may "push" electrical charge into the positive terminal of battery 108, thereby degrading battery 108. If battery housing 109 is case neutral, electrical charge may accumulate on at least a portion of the inner surface of battery housing 109 and cause depletion of the battery cells, voltaic cells, battery materials or chemistry housed within battery housing 109, thereby degrading battery 108. If battery housing 109 is case positive or case negative, IMD 100 may "shunt" electrical charge, e.g., the electrical charge may accumulate on at least a portion of the inner surface of battery housing 109 and cause depletion of the battery cells, voltaic cells, battery materials or chemistry housed within battery housing 109, thereby degrading battery 108. For example, if IMD 100 provides stimulation with a voltage high enough, the case (e.g., battery housing 109) may be driven well above the cathode potential resulting in electrolyte reduction at the case wall.

If battery housing 109 is case negative, secondary electrode 106 is no longer floating and is connected to "ground," e.g., the lowest electrical potential (e.g., voltage) of the system or circuit, which is the negative terminal of battery 108 in the example shown when there is a direct electrical path between secondary electrode 106 and battery housing 109. As such, the flow of current between primary electrode 104 and secondary electrode 106 (through patient tissue and/or fluids) reverses, e.g., primary electrode 104 is the anode and secondary electrode 106 is the cathode, e.g., an electrical current sink or "return electrode" for an electrical stimulation signal of IMD 100. IMD 100 may then "push" electrical charge into the negative terminal of battery 108, which is the normal operating condition for battery 108.

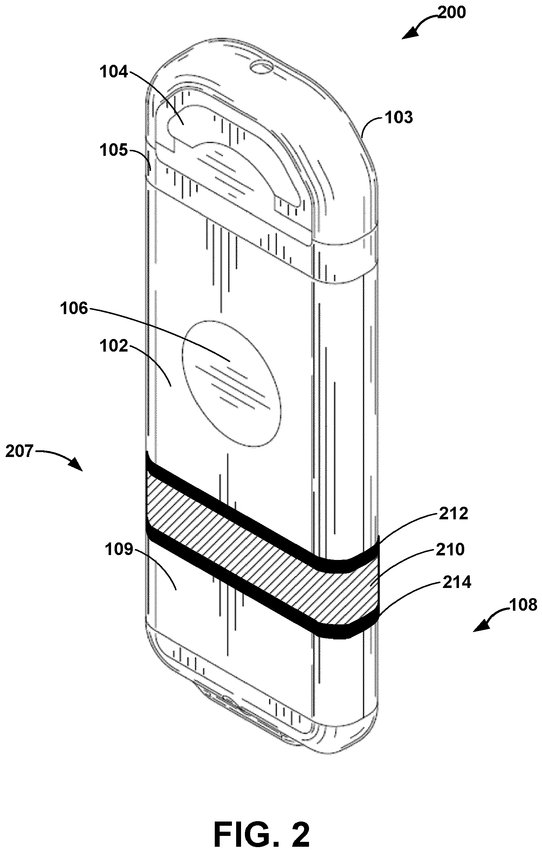

FIG. 2 is a schematic diagram illustrating an example medical device 200, also referred to as IMD 200. IMD 200 includes a device housing 102 containing one or more electrical components therein, such as circuitry, antennas, electrode connections, or the like. In some examples, IMD 200 may be a leadless neurostimulation device configured for delivering neurostimulation therapy, and IMD 200 may be substantially similar to IMD 100 described above, except that IMD 200 includes insulator 207.

In the example shown, insulator 207 may be substantially similar to attachment 107 described above, except that insulator 207 includes an electrical insulator configured to electrically isolate, separate, and/or increase the electrical resistance between device housing 102 and battery housing 109. Insulator 207 may comprise any suitable material configured to both attach and increase the electrical resistance between device housing 102 and battery housing 109. For example, insulator 207 may comprise a ceramic, such as sapphire. In the example shown, insulator 207 comprises ceramic 210 and attachment portions 212 and 214. Attachment portions 212 and 214 may comprise titanium weld ferrules that may be brazed or diffusion bonded to ceramic 210 and are configured to attach device housing 102 and battery housing 109 to ceramic 210, respectively. Attachment portions 212 and 214 may form a hermetic seal between device housing 102 and ceramic 210, and between battery housing 109 and ceramic 210, respectively. In some examples, ceramic 210 may be a ceramic ring and may extend about the circumference or outer dimension of device housing 102 and/or battery 108. In some examples, an outer surface of insulator 210, e.g., any or all of ceramic 210 and attachment portions 212, 214, may comprise external surfaces that are coplanar with at least a portion of device housing 102 and/or battery 108. In some examples, insulator 207 may define an inner lumen between device housing 102 and battery housing 109 and that may enclose a volume when battery housing 109 and device housing 102 are attached via insulator 207.

In some examples, insulator 207 may be configured to house one or more components within the lumen and/or volume. Examples of the one or more components within the lumen and/or volume includes electrical connectors or conductors between battery 108 and one or more electrical components housed within device housing 102, circuitry, one or more antennas such as a Bluetooth^{®} or other wireless antenna and associated communication circuitry, or the like. For example, ceramic 210 may be at least partially transparent to electromagnetic waves at radio frequencies, e.g., RF communication waves, and insulator 207 may be configured to house communication components such as a Bluetooth^{®} antenna. In some examples, IMD 200 is configured to improve wireless communication of an implantable medical device, e.g., via RF transparent ceramic 210.

As discussed above with respect to FIG. 1, and similar functionality with respect to FIG. 2, battery housing 109 may be shunted to device housing 102 via tissue and/or fluids of the patient, e.g., when IMD 200 is implanted. In some examples, IMD 200 may include a biocompatible electrical insulator, such as a parylene coating disposed on the outer surfaces of device housing 102, header unit 103, battery housing 109, and optionally on insulator 207, with the exception of the surfaces of primary and secondary electrodes 104 and 106. The biocompatible electrical insulator may be configured to electrically isolate and/or separate battery housing 109 from device housing 102, or to otherwise increase an electrical resistance between battery housing 109 and device housing 102.

In the example shown, battery housing 109 may be electrically isolated from device housing 102 via insulator 207 and a biocompatible electrical insulator, e.g., removing the direct or low resistance electrical paths between battery housing 109 and device housing 102. Electrically isolating battery housing 109 from device housing 102 (e.g., and from electrodes 104, 106) may enable IMD 200 to be operated in a biphasic mode, e.g., a mode comprising two phases of electrical stimulation. The two modes may be a first mode or "charge" mode in which IMD 200 causes a current to flow through tissue and/or fluid of the patient between electrodes 104, 106 in a first direction and a second mode or "active recharge" mode in which IMD 200 causes a current to flow through the tissue and/or fluid between electrodes 104, 106 in a second direction opposite the first direction.

For example, whether battery housing 109 is a terminal of battery 108, or is case positive, case neutral, or case negative, IMD 200 may be configured to reverse the current flow through tissue and/or fluids of the patient between primary and secondary electrodes 104 and 106 without negative effects on battery 108 and/or the efficacy of the stimulation. For example, IMD 200 may provide electrical stimulation with primary electrode 104 as a cathode and secondary electrode 106 as an anode. That is, circuitry with IMD 200 may be configured to connect (e.g., via a switch) primary electrode 104 to a negative terminal of battery 108 and to connect secondary electrode 106 to a positive terminal of battery 108 to cause a current to flow through tissue and/or fluid of the patient between electrodes 106, 104 and delivery stimulation. The current flow through the tissue and/or fluids may change and/or charge the tissue and/or fluids, and a current may flow through the tissue and/or fluid as the charged tissue and/or fluid discharges upon IMD 200 shutting off the flow of current, e.g., disconnecting electrodes 104, 106 from battery 108 terminals. As such, IMD 200 may provide passive recharge by removing the flow of current for a period of time and allowing the tissue and/or fluid to discharge localized buildup of charge.

In other examples, IMD 200 may be configured to provide active recharge, e.g., to switch the direction of the flow of current through the tissue and/or fluid and thereby removing the buildup of charge in the tissue and/or fluid. For example, after connecting primary electrode 104 to a negative terminal of battery 108 and connecting secondary electrode 106 to a positive terminal of battery 108 to cause a current to flow through tissue and/or fluid of the patient between electrodes 106, 104 in a first direction, IMD 200 may connect primary electrode 104 to the positive terminal of battery 108 and connect secondary electrode 106 to the negative terminal of battery 108 to cause a current to flow through tissue and/or fluid of the patent between electrodes 106, 104 in a second, opposite direction, for a period of time.

IMD 200 may be configured to provide passive or active recharge with battery housing 109 at case positive, case neutral, or case negative. In some examples, although not required, IMD 100 may be configured to provide only passive recharge with battery housing 109 at case negative, e.g., because secondary electrode 106 is shunted to battery housing 109 and the negative terminal of battery 108 and cannot be switched to the positive terminal. In some examples, the biocompatible electrical insulator disposed on surfaces of IMD 200 may be at least partially compromised, e.g., include and/or develop nicks, cuts, voids, or the like, reducing the efficacy of electrically isolating battery housing 109 from device housing 102 and/or electrodes 104, 106. Battery housing 109 may be configured to be case negative, e.g., even when electrically isolated from device housing 102 and/or electrodes 104, 106.

In some examples, IMD 100 and/or IMD 200 may be a constant current device, e.g., as opposed to constant voltage device. For example, IMD 200 may be configured to provide electrical stimulation by controlling the stimulation circuit to have a constant current rather than apply a constant voltage to one or both of electrodes 104, 106.

In some examples, an IMD may include an externalized battery that includes a polymer enclosure (not shown). For example, an IMD 100 and/or IMD 200 may include an externalized battery that is substantially similar to battery 108, except that there is a polymer enclosure disposed between battery housing 109 and the one or more battery cells, the one or more voltaic cells, the battery materials and/or the battery chemistry of the battery. In some examples, a battery including a polymer enclosure may allow for an increase in battery capacity by reducing the enclosure thickness, e.g., the battery enclosure wall thickness. In examples in which IMD 100 includes a battery including a polymer enclosure, battery housing 109 may no longer be a terminal of the battery and may enable IMD 100 to provide active recharging.

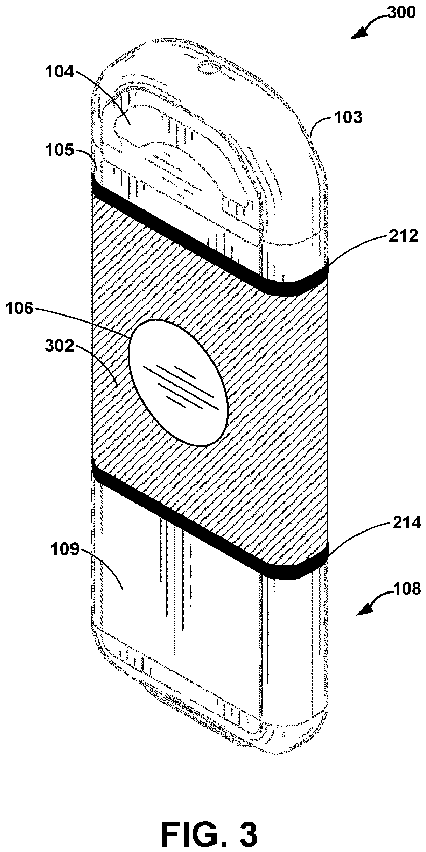

FIG. 3 is a schematic diagram illustrating an example medical device 300, also referred to as IMD 300. IMD 300 includes a device housing 302 comprising an electrically insulating material (e.g., a ceramic such as sapphire) and is configured to contain one or more electrical components therein, such as circuitry, antennas, electrode connections, or the like. In some examples, IMD 300 may be a leadless neurostimulation device configured for delivering neurostimulation therapy, and IMD 300 may be substantially similar to IMDs 100 and 200 described above, except that IMD 300 includes electrically insulating device housing 302 rather than device housing 102 and does not include ceramic 210. Device housing 302 may be substantially similar to device housing 102, except that device housing 302 is electrically insulating.

In the example shown, attachment portions 212 and 214 may comprise titanium weld ferrules that may be brazed or diffusion bonded to device housing 302 and are configured to attach header unit 103 and battery housing 109 to device housing 302, respectively. Secondary electrode 106 may be brazed or diffusion bonded to device housing 302, e.g., via weld ferrules (not shown). In other examples, secondary electrode 106 may be attached to device housing 302 via any suitable attachment method or material, e.g., an adhesive, a mechanical coupling, or the like. In some examples, an outer surface of device housing 302, e.g., any or all of device housing 302 and attachment portions 212, 214, may comprise external surfaces that are coplanar with at least a portion of device housing 102 and/or battery 108.

As discussed above, battery housing 109 may be shunted to device housing 102 of IMD 100 or IMD 200 via tissue and/or fluids of the patient, e.g., when IMD 200 is implanted. Similarly, battery housing 109 may be shunted to one or both of electrodes 104, 106 via tissue and/or fluids of the patient, albeit with an increased path length through the tissue and/or fluids because device housing 302 comprises and electrically insulating material. In some examples, IMD 300 may include a biocompatible electrical insulator, such as a parylene coating disposed on the outer surfaces of device housing 302, header unit 103, and battery housing 109, with the exception of the surfaces of primary and secondary electrodes 104 and 106. The biocompatible electrical insulator may be configured to electrically isolate and/or separate battery housing 109 from electrodes 104, 106, or to otherwise increase an electrical resistance between battery housing 109 and electrodes 104, 106.

In some examples, device housing 302 may be configured to improve wireless transfer of power between IMD 300 and an external power source, e.g., external to the patient within which IMD 300 is implanted. For example, IMD 300 (or IMD 200 or IMD 100) may comprise a wireless power receiver coil configured to receive power wirelessly to recharge battery 108, e.g., within device housing 302 (or device housing 102). With externalized battery 108. The wireless power receiver coil of IMD 300 may be smaller and have a reduced aperture relative to an IMD having a battery within its device housing, e.g., the wireless power receiver coil may extend substantially the entire width and length of the device housing. Because device housing 302 (or device housing 102) may have a reduced length (or width), the aperture of a wireless power receiver coil may be correspondingly reduced. Device housing 302 may be substantially transparent to the electromagnetic fields and/or waves used for transferring power, and may increase the efficiency of the power transfer through device housing 302. In some examples, device housing 302 may compensate for the smaller aperture of the wireless power receiver coil, and in other examples device housing 302 may enable an increase and/or improvement of the amount and/or rate of power transferred wirelessly via the wireless power receiver coil housed within device housing 302.

IMD 300 may be configured to provide passive or active recharge with battery housing 109 at case positive, case neutral, or case negative. In some examples, the biocompatible electrical insulator disposed on surfaces of IMD 300 may be at least partially compromised, e.g., include and/or develop nicks, cuts, voids, or the like, reducing the efficacy of electrically isolating battery housing 109 from electrodes 104, 106. Battery housing 109 may be configured to be case negative, e.g., even when electrically isolated from electrodes 104, 106.

In some examples, device housing 302 may be configured to house one or more antennas such as a Bluetooth^{®} or other wireless antenna and associated communication circuitry, or the like. For example, device housing 302 may be at least partially transparent to electromagnetic waves at radio frequencies, e.g., RF communication waves, and device housing 302 may be configured to house communication components such as a Bluetooth^{®} antenna. In some examples, IMD 300 is configured to improve wireless communication of an implantable medical device, e.g., via RF transparent device housing 302.

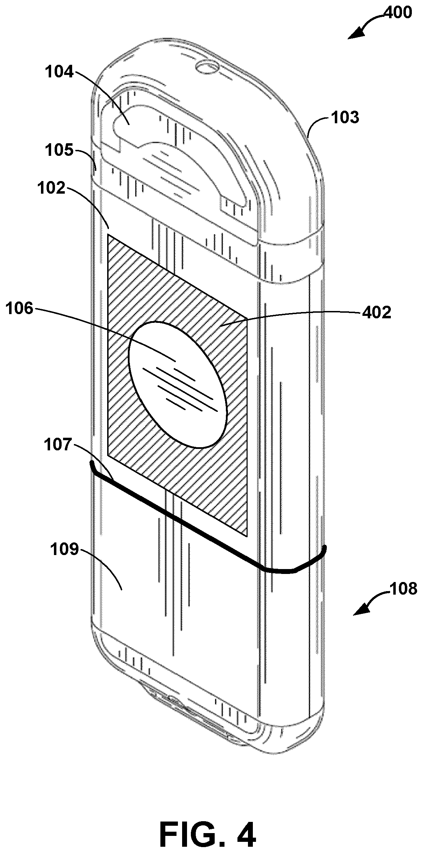

FIG. 4 is a schematic diagram illustrating an example medical device 400, also referred to as IMD 400. IMD 400 includes a device housing 102, a portion of which may comprise electrically insulating portion 402 comprising an electrically insulating material (e.g., a ceramic such as sapphire). In some examples, IMD 400 may be a leadless neurostimulation device configured for delivering neurostimulation therapy, and IMD 400 may be substantially similar to IMD 300 described above, except that IMD 400 includes electrically insulating portion 402 rather than device housing 302 and attachment 107 rather than attachment portions 212, 214.

Similar to device housing 302, electrically insulating portion 402 may be configured to improve wireless transfer of power and electrically isolate secondary electrode from housing 102. In some examples, secondary electrode 106 may be attached to electrically insulating portion 402 similarly to device housing 302 as described above. In other examples, secondary electrode 106 may be located and/or attached to a surface of housing 102, e.g., on the opposite side of IMD 400. Similar to IMDs 100-300, at least a portion, or all, of the external surfaces of IMD 400 (e.g., housing 102, header unit 103, battery housing 109, and electrically insulating portion 402) may be coated and/or otherwise in contact with a biocompatible electrical insulator (e.g., parylene), with the exception of the surface of electrodes 104, 106.

IMD 400 may be configured to provide passive or active recharge with battery housing 109 at case positive, case neutral, or case negative. In some examples, the biocompatible electrical insulator disposed on surfaces of IMD 400 may be at least partially compromised, e.g., include and/or develop nicks, cuts, voids, or the like, reducing the efficacy of electrically isolating battery housing 109 from device housing 102 and/or electrodes 104, 106. Battery housing 109 may be configured to be case negative, e.g., even when electrically isolated from device housing 102 and/or electrodes 104, 106.

In some examples, device housing 102 may be configured to house one or more antennas such as a Bluetooth^{®} or other wireless antenna and associated communication circuitry, or the like. For example, electrically insulating portion 402 may be at least partially transparent to electromagnetic waves at radio frequencies, e.g., RF communication waves, and device housing 102 may be configured to house communication components such as a Bluetooth^{®} antenna. In some examples, IMD 400 is configured to improve wireless communication of an implantable medical device, e.g., via RF transparent electrically insulating portion 402.

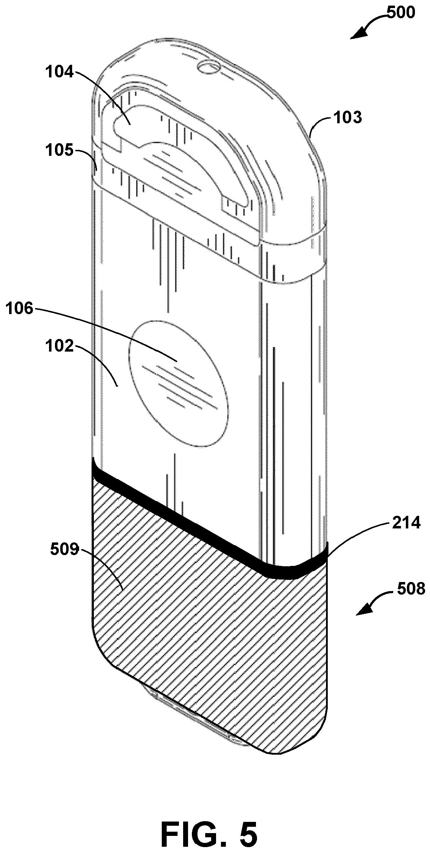

FIG. 5 is a schematic diagram illustrating an example medical device 500, also referred to as IMD 500. IMD 500 includes externalized battery 508 which may be substantially similar to externalized battery 108 except that externalized battery 508 includes an electrically insulating battery housing 509 (e.g., a ceramic such as sapphire) rather than battery housing 109. In some examples, IMD 500 may be a leadless neurostimulation device configured for delivering neurostimulation therapy, and IMD 500 may be substantially similar to IMD 200 described above, except that IMD 500 includes externalized battery 508 rather than externalized battery 108. IMD 500 may be configured to provide passive or active recharge.

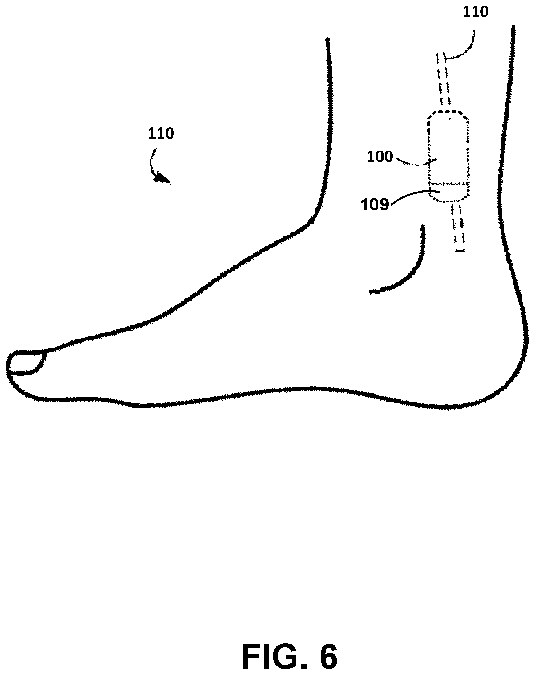

FIG. 6 is a conceptual diagram illustrating a leg of a patient having IMD 100 implanted therein near a tibial nerve 110. In the example of FIG. 2, IMD 100 is implanted near tibial nerve 110 in leg 111 of a patient. For example, IMD 100 may deliver neurostimulation to the patient to manage bladder dysfunction, such as overactive bladder, urgency, or urinary incontinence.

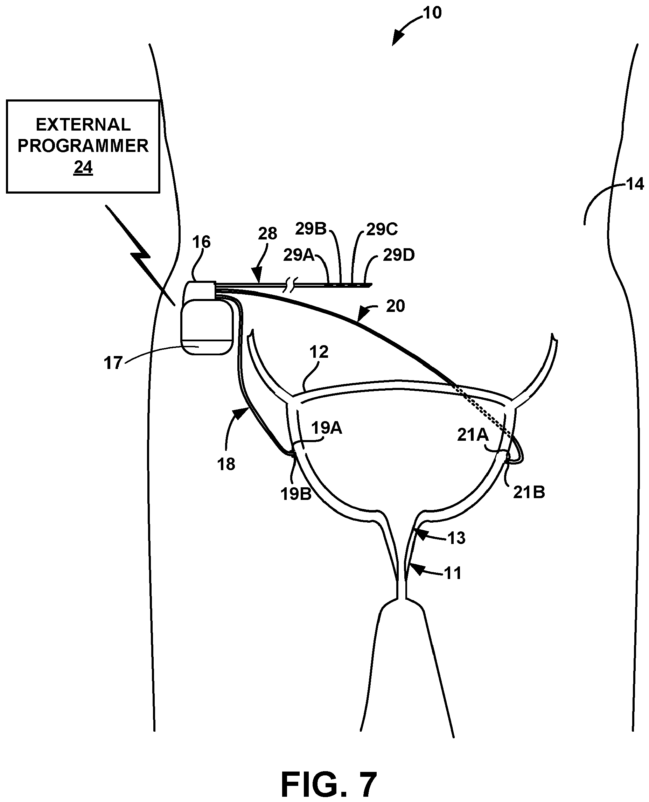

FIG. 7 is a conceptual diagram illustrating an example system 10 that manages delivery of neurostimulation to patient 14 to manage bladder dysfunction, such as overactive bladder, urgency, or urinary incontinence. Therapy system 10 includes IMD 16 (e.g., an example medical device), which is coupled to leads 18, 20, and 28 and sensor 22. In some examples, IMD 16 may be substantially similar to any one of IMDs 100, 200, 300, 400, or 500, except that IMD 16 may be a leaded neurostimulation device rather than a leadless neurostimulation device. For example, IMD 16 may include externalized battery 17, which may be substantially similar to externalized battery 108 and/or 508 described above. System 10 also includes an external programmer 24, which is configured to communicate with IMD 16 via wireless communication. IMD 16 generally operates as a therapy device that delivers electrical neurostimulation to, for example, a target tissue site proximate a tibial nerve, a spinal nerve, a sacral nerve, a pudendal nerve, dorsal genital nerve, an inferior rectal nerve, a perineal nerve, or other pelvic nerves, or branches of any of the aforementioned nerves. IMD 16 provides electrical stimulation to patient 14 by generating and delivering a programmable electrical stimulation signal (e.g., in the form of electrical pulses or an electrical waveform) to a target a therapy site near lead 28 and, more particularly, near electrodes 29A-29D (collectively referred to as "electrodes 29") disposed proximate to a distal end of lead 28.

IMD 16 may be surgically implanted in patient 14 at any suitable location within patient 14, such as near the pelvis. In some examples, IMD 16 may be implanted in a subcutaneous location in the side of the lower abdomen or the side of the lower back or upper buttocks. IMD 16 has a biocompatible housing, which may be formed from titanium, stainless steel, a liquid crystal polymer, or the like. The proximal ends of leads 18, 20, and 28 are both electrically and mechanically coupled to IMD 16 either directly or indirectly, e.g., via respective lead extensions. Electrical conductors disposed within the lead bodies of leads 18, 20, and 28 electrically connect sense electrodes (e.g., electrodes 19A, 19B, 21A, and 21B) and stimulation electrodes, such as electrodes 29, to sensing circuitry and a stimulation delivery circuitry (e.g., a stimulation generator) within IMD 16. In the example of FIG. 1C, leads 18 and 20 carry electrodes 19A, 19B (collective referred to as "electrodes 19") and electrodes 21A, 21B (collectively referred to as "electrodes 21"), respectively. Electrodes 19 and 21 may be positioned for sensing an impedance of bladder 12, which may increase as the volume of urine within bladder 12 increases.

External programmer 24 may receive user input identifying a voiding event, perceived level of fullness, or the like. The user input may be in the form of a voiding journal analyzed by external programmer 24 or IMD 16 or individual user inputs associated with respective voiding events, leakage, or any other event related to a phase of the physiological cycle. External programmer 24 and/or IMD 16 may use this user input to generate estimated fill cycles and determine when to exit hibernation mode and deliver stimulation and when to withhold stimulation and enter hibernation mode. In other words, one or more physiological markers may be identified from user input and utilized for determining when to enter and exit hibernation mode. The user input may be in addition to or instead of sensors such as electrodes 19A and 21A for detecting a physiological marker.

One or more medical leads, e.g., leads 18, 20, and 28, may be connected to IMD 16 and surgically or percutaneously tunneled to place one or more electrodes carried by a distal end of the respective lead at a desired nerve or muscle site, e.g., one of the previously listed target therapy sites such as a tissue site proximate a tibial, spinal, sacral, or pudendal nerve. For example, lead 28 may be positioned such that electrodes 29 deliver electrical stimulation to a tibial, spinal, sacral, or pudendal nerve to reduce a frequency and/or magnitude of contractions of bladder 12. Additional electrodes of lead 28 and/or electrodes of another lead may provide additional stimulation therapy to other nerves or tissues as well. In FIG. 7, leads 18 and 20 are placed proximate to an exterior surface of the wall of bladder 12 at first and second locations, respectively. In other examples of therapy system 10, IMD 16 may be coupled to more than one lead that includes electrodes for delivery of electrical stimulation to different stimulation sites within patient 14, e.g., to target different nerves.

The illustrated numbers and configurations of leads 18, 20, and 28 and electrodes carried by leads 18, 20, and 28 are merely exemplary. Other configurations, e.g., numbers and positions of leads and electrodes are also contemplated. For example, in other implementations, IMD 16 may be coupled to additional leads or lead segments having one or more electrodes positioned at different locations proximate the spinal cord or in the pelvic region of patient 14. The additional leads may be used for delivering different stimulation therapies or other electrical stimulations to respective stimulation sites within patient 14 or for monitoring at least one physiological marker of patient 14.

In some examples, IMD 16 delivers electrical stimulation based on a stimulation program to at least one of a tibial nerve, spinal nerve (e.g., a sacral nerve), a pudendal nerve, dorsal genital nerve, an inferior rectal nerve, or a perineal nerve to provide a therapeutic effect that reduces or eliminates a dysfunctional state such as overactive bladder. The desired therapeutic effect may be an inhibitory physiological response related to voiding of patient 14, such as a reduction in bladder contraction frequency by a desired level or degree (e.g., percentage).

The stimulation program may define various parameters of the stimulation waveform and electrode configuration which result in a predetermined stimulation intensity being delivered to the targeted nerve or tissue. In some examples, the stimulation program defines parameters for at least one of a current or voltage amplitude of the stimulation signal, a frequency or pulse rate of the stimulation, the shape of the stimulation waveform, a duty cycle of the stimulation, a pulse width of the stimulation, and/or the combination of electrodes 29 and respective polarities of the subset of electrodes 29 used to deliver the stimulation. Together, these stimulation parameter values may be used to define the stimulation intensity (also referred to herein as a stimulation intensity level). In some examples, if stimulation pulses are delivered in bursts, a burst duty cycle also may contribute to stimulation intensity. Also, independent of intensity, a particular pulse width and/or pulse rate may be selected from a range suitable for causing the desired therapeutic effect after stimulation is terminated and, optionally, during stimulation. In addition, as described herein, a period during which stimulation is delivered may include on and off periods (e.g., a duty cycle or bursts of pulses) where even the short inter-pulse durations of time when pulses are not delivered are still considered part of the delivery of stimulation. For example, IMD 16 may not enter the hibernation mode during the short inter-pulse durations of time when pulses are not delivered. For example, the short inter-pulse duration of time when pulses are not delivered during an on-cycle is still considered part of delivery of stimulation, but during an off-cycle, IMD 16 may be considered as not delivering stimulation.

Although the above is described with respect to stimulation that IMD 16 may deliver, in one or more examples, IMDs 100, 200, 300, 400, and 500 may be configured to deliver therapy similar to examples described above. However, in some examples, IMDs 100, 200, 300, 400, and 500 may deliver stimulation for a limited amount of time, and then remain in hibernation mode for an extended period of time (e.g., deliver a plurality of stimulation pulses for 30 minutes, and be in hibernation mode for 23.5 hours, 47.5 hours, etc.).

System 10 may also include an external programmer 24, as shown in FIG. 7. External programmer 24 may be a clinician programmer or patient programmer. In some examples, external programmer 24 may be a wearable communication device, with a therapy request input integrated into a key fob or a wristwatch, handheld computing device, smart phone, computer workstation, or networked computing device. External programmer 24 may include a user interface that is configured to receive input from a user (e.g., patient 14, a patient caretaker, or a clinician). In some examples, the user interface includes, for example, a keypad and a display, which may for example, be a liquid crystal display (LCD) or light emitting diode (LED) display. The keypad may take the form of an alphanumeric keypad or a reduced set of keys associated with particular functions. External programmer 24 may additionally or alternatively include a peripheral pointing device, such as a mouse, via which a user may interact with the user interface. In some examples, a display of external programmer 24 may include a touch screen display, and a user may interact with external programmer 24 via the display. It should be noted that the user may also interact with external programmer 24 and/or IMD 16 remotely via a networked computing device. In some examples, external programmer 24 may be configured to interoperate with any of IMDs 100, 200, 300, 400, or 500, e.g., leadless IMDs.

A user, such as a physician, technician, surgeon, electrophysiologist, or other clinician, may also interact with external programmer 24 or another separate programmer (not shown), such as a clinician programmer, to communicate with IMD 16. Such a user may interact with a programmer to retrieve physiological or diagnostic information from IMD 16. The user may also interact with a programmer to program IMD 16, e.g., select values for the stimulation parameter values with which IMD 16 generates and delivers stimulation and/or the other operational parameters of IMD 16, such as magnitudes of stimulation energy, user requested periods for stimulation or periods to prevent stimulation, or any other such user customization of therapy. As discussed herein, the user may also provide input to external programmer 24 indicative of physiological events such as bladder fill level perception and void events.

For example, the user may use a programmer to retrieve information from IMD 16 regarding the contraction frequency of bladder 12 and/or voiding events. As another example, the user may use a programmer to retrieve information from IMD 16 regarding the performance or integrity of IMD 16 or other components of system 10, such as leads 18, 20, and 28, or a power source of IMD 16. In some examples, this information may be presented to the user as an alert if a system condition that may affect the efficacy of therapy is detected.

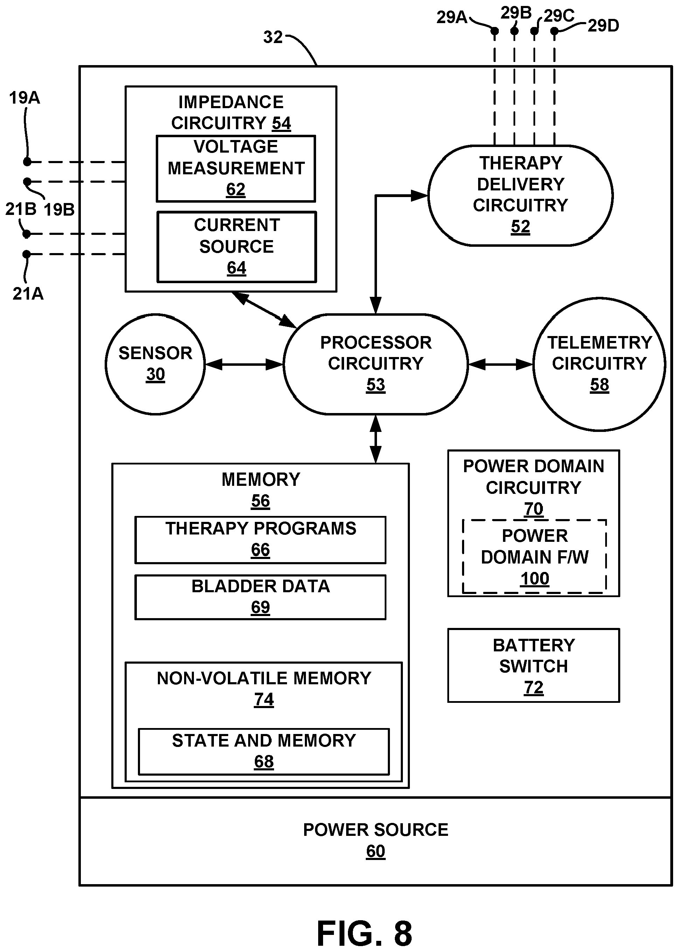

FIG. 8 is a block diagram illustrating an example configuration of an IMD. As shown in FIG. 8, IMD 32, which may be an example of leadless IMDs 100-500 or leaded IMD 16, includes sensor 22, processor circuitry 53, stimulation circuitry 52, impedance circuitry 54, memory 56, telemetry circuitry 58, hibernation control circuitry 70, isolation interface circuitry 72, and power source 60. In other examples, IMD 32 may include a greater or fewer number of components. For example, in the case where IMD 32 represents IMDs 100-500, IMD 32 does not include leads, but rather electrodes are disposed on the surface of IMD 32.

In general, IMD 32 may comprise any suitable arrangement of hardware, alone or in combination with software and/or firmware, to perform the techniques attributed to IMD 32 and components of IMD 32. In various examples, IMD 32 may include one or more processors, such as one or more microprocessors, digital signal processors (DSPs), application specific integrated circuits (ASICs), field programmable gate arrays (FPGAs), or any other equivalent integrated or discrete logic circuitry, as well as any combinations of such components. IMD 32 also, in various examples, may include a memory 56, such as random access memory (RAM), read only memory (ROM), programmable read only memory (PROM), erasable programmable read only memory (EPROM), electronically erasable programmable read only memory (EEPROM), flash memory, comprising executable instructions for causing the one or more processors to perform the actions attributed to them. Moreover, although processor circuitry 53, stimulation circuitry 52, impedance circuitry 54, and telemetry circuitry 58 are described as separate circuitry, in some examples, processor circuitry 53, stimulation circuitry 52, impedance circuitry 54, and telemetry circuitry 58 are functionally integrated. In some examples, processor circuitry 53, stimulation circuitry 52, impedance circuitry 54, and telemetry circuitry 58 correspond to individual hardware units, such as microprocessors, ASICs, DSPs, FPGAs, or other hardware units. In further examples, any of processor circuitry 53, stimulation circuitry 52, impedance circuitry 54, and telemetry circuitry 58 may correspond to multiple individual hardware units such as microprocessors, ASICs, DSPs, FPGAs, or other hardware units.

Memory 56 stores therapy programs 66 that specify stimulation parameter values and electrode combinations for the electrical stimulation provided by IMD 32. Therapy programs 66 may also store information regarding determining and using physiological parameters, information regarding physiological cycles and/or dysfunctional states, or any other information required by IMD 32 to deliver stimulation therapy based on one or more physiological parameters of patient 14. In some examples, memory 56 also stores bladder data 69, which processor circuitry 53 may use for controlling the timing of the delivery of the electrical stimulation (e.g., phases of physiological cycles that define when to deliver and withhold stimulation). For example, bladder data 69 may include threshold values or baseline values for at least one of bladder impedance, bladder pressure, afferent nerve signals, bladder contraction frequency, or external urinary sphincter EMG templates for use as physiological markers for an associated physiological cycle. Bladder data 69 may also include timing information and physiological markers associated with physiological events, such as a voiding event.

Memory 56 may also store state and memory information 68. For example, when IMD 32 is about to enter a particular mode, processor circuitry 53 may store certain state and memory information in state and memory information 68. State and memory information 68 may then be accessed by processor circuitry 53 when IMD 32 is exiting the particular mode, which processor circuitry may use to restore IMD 32 to a normal operative state. State and memory information may include time information indicative of when the hibernation mode is being entered, a desired exit time, length of time of last therapy delivery, or the like. State and memory information may also include information regarding sensor acquisition and therapy sequences. State and memory information may include algorithm states and data for assessments that are underway, such as bladder filling calculations or therapy titration calculations. State and memory information may also include diagnostic data for the device state, such as battery voltage, electrode impedances, and/or telemetry logs.

Information related to sensed bladder contractions, bladder impedance and/or posture of patient 14 may be stored in bladder data 69. Bladder data 69 may be retrieved by a user, and/or used by processor circuitry 53 for adjustment of stimulation parameters (e.g., amplitude, pulse width, and pulse rate). In some examples, memory 56 includes separate memories for storing instructions, electrical signal information, stimulation programs 66, state and memory information 68, and bladder data 69. In some examples, processor circuitry 53 selects new stimulation parameters for a therapy program 66 or new stimulation program from stimulation programs 66 to use in the delivery of the electrical stimulation based on patient input or sensor signals. In some examples, processor circuitry 53 may use bladder data 69 to determine efficacy of a therapy program and may adjust periods of stimulation and periods of withholding of stimulation and the associated operational mode and hibernation mode based on the determined efficacy of the therapy program.

Generally, stimulation circuitry 52 generates and delivers electrical stimulation under the control of processor circuitry 53. As used herein, controlling the delivery of electrical stimulation may also include controlling the termination of stimulation to achieve the different stimulation and non-stimulation phases. In some examples, processor circuitry 53 controls stimulation circuitry 52 by accessing memory 56 to selectively access and load at least one of stimulation programs 66 to stimulation circuitry 52. For example, in operation, processor circuitry 53 may access memory 56 to load one of stimulation programs 66 to stimulation circuitry 52. In other examples, stimulation circuitry 52 may access memory 56 and load one of the stimulation programs 66.

By way of example, processor circuitry 53 may access memory 56 to load one of stimulation programs 66 to stimulation circuitry 52 for delivering the electrical stimulation to patient 14. A clinician or patient 14 may select a particular one of stimulation programs 66 from a list using a programming device, such as external programmer 24 or a clinician programmer. Processor circuitry 53 may receive the selection via telemetry circuitry 58. Stimulation circuitry 52 delivers the electrical stimulation to patient 14 according to the selected program for an extended period of time, such as minutes, hours, days, weeks, or until patient 14 or a clinician manually stops or changes the program.

Therapy delivery circuitry 52 delivers electrical stimulation according to stimulation parameters. In some examples, stimulation circuitry 52 delivers electrical stimulation in the form of electrical pulses. In such examples, relevant stimulation parameters may include a voltage amplitude, a current amplitude, a pulse rate, a pulse width, a duty cycle, or the combination of electrodes 29 that stimulation circuitry 52 uses to deliver the stimulation signal. In other examples, stimulation circuitry 52 delivers electrical stimulation in the form of continuous waveforms. In such examples, relevant stimulation parameters may include a voltage or current amplitude, a frequency, a shape of the stimulation signal, a duty cycle of the stimulation signal, or the combination of electrodes 29 stimulation circuitry 52 uses to deliver the stimulation signal.

In the example of FIG. 8, stimulation circuitry 52 drives electrodes on a single lead 28. Specifically, stimulation circuitry 52 delivers electrical stimulation to tissue of patient 14 via selected electrodes 29A-29D carried by lead 28. A proximal end of lead 28 extends from the housing of IMD 32 and a distal end of lead 28 extends to a target therapy site, such as a tibial nerve, a spinal nerve (e.g., an S3 nerve), or a therapy site within the pelvic floor, such as tissue sites proximate a sacral nerve, a pudendal nerve, a dorsal genital nerve, an inferior rectal nerve, a perineal nerve, a hypogastric nerve, a urinary sphincter, or any combination thereof. In other examples, stimulation circuitry 52 may deliver electrical stimulation with electrodes on more than one lead and each of the leads may carry one or more electrodes. The leads may be configured as an axial lead with ring electrodes or segmented electrodes and/or paddle leads with electrode pads arranged in a two-dimensional array. The electrodes may operate in a bipolar or multi-polar configuration with other electrodes, or may operate in a unipolar configuration referenced to an electrode carried by the device housing or "can" of IMD 32. For example, IMD 32 may be configured to operate in a bipolar configuration with externalized battery 60 (e.g., power source 60 described below) at case negative.

Telemetry circuitry 58 includes any suitable hardware, firmware, software or any combination thereof for communicating with another device, such as external programmer 24 (FIG. 7). Under the control of processor circuitry 53, telemetry circuitry 58 may receive downlink telemetry, e.g., patient input, from and send uplink telemetry, e.g., an alert, to external programmer 24 with the aid of an antenna, which may be internal and/or external. Processor circuitry 53 may provide the data to be uplinked to external programmer 24 and the control signals for the telemetry circuit within telemetry circuitry 58, and receive data from telemetry circuitry 58.

Generally, processor circuitry 53 may control telemetry circuitry 58 to exchange information with external programmer 24 and/or another device external to IMD 32. Processor circuitry 53 may transmit operational information and receive stimulation programs or stimulation parameter adjustments via telemetry circuitry 58. Also, in some examples, IMD 32 may communicate with other implanted devices, such as stimulators, control devices, or sensors, via telemetry circuitry 58.

Power source 60 delivers operating power to the components of IMD 32. Power source 60 may include an externalized battery substantially similar to externalized battery 108 or 508 described above and a power generation circuit to produce the operating power. In some examples, the externalized battery may be rechargeable to allow extended operation. Recharging may be accomplished through proximal inductive interaction between an external charger and an inductive charging coil (e.g., a wireless power receiver coil) within IMD 32. In other examples, an external inductive power supply may transcutaneously power IMD 32 whenever electrical stimulation is to occur.

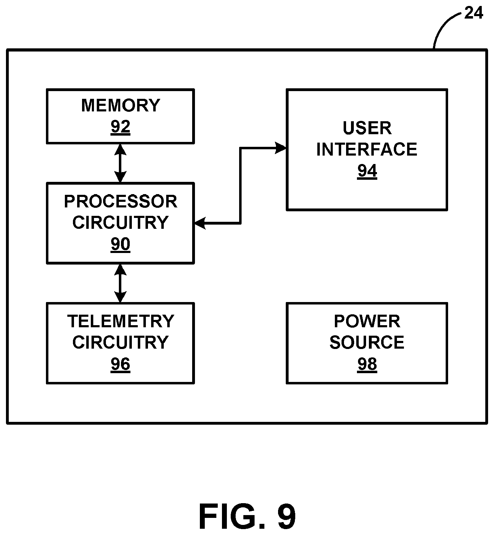

FIG. 9 is a block diagram illustrating an example configuration of an external programmer 24. While external programmer 24 may generally be described as a hand-held computing device, external programmer 24 may be a notebook computer, a smart phone, or a workstation, for example. As illustrated in FIG. 9, external programmer 24 may include a processor circuitry 90, memory 92, user interface 94, telemetry circuitry 96, and power source 98. Memory 92 may store program instructions that, when executed by processor circuitry 90, cause processor circuitry 90 and external programmer 24 to provide the functionality ascribed to external programmer 24 throughout this disclosure.

In general, external programmer 24 comprises any suitable arrangement of hardware, alone or in combination with software and/or firmware, to perform the techniques attributed to external programmer 24, and processor circuitry 90, user interface 94, and telemetry circuitry 96 of external programmer 24. In various examples, external programmer 24 may include one or more processors, such as one or more microprocessors, DSPs, ASICs, FPGAs, or any other equivalent integrated or discrete logic circuitry, as well as any combinations of such components. External programmer 24 also, in various examples, may include a memory 92, such as RAM, ROM, PROM, EPROM, EEPROM, flash memory, a hard disk, a CD-ROM, comprising executable instructions for causing the one or more processors to perform the actions attributed to them. Moreover, although processor circuitry 90 and telemetry circuitry 96 are described as separate circuitry, in some examples, processor circuitry 90 and telemetry circuitry 96 are functionally integrated. In some examples, processor circuitry 90 and telemetry circuitry 96 and telemetry circuitry 58 correspond to individual hardware units, such as microprocessors, ASICs, DSPs, FPGAs, or other hardware units. In other examples, any of processor circuitry 90 and telemetry circuitry 96 and telemetry circuitry 58 may correspond to multiple individual hardware units, such as microprocessors, ASICs, DSPs, FPGAs, or other hardware units.

Memory 92 may store program instructions that, when executed by processor circuitry 90, cause processor circuitry 90 and external programmer 24 to provide the functionality ascribed to external programmer 24 throughout this disclosure. In some examples, memory 92 may further include program information, e.g., stimulation programs defining the neurostimulation, similar to those stored in memory 56 of IMD 32. The stimulation programs stored in memory 92 may be downloaded into memory 56 of IMD 32.

In certain examples, the system includes a user interface 94 that allows patient 14 to provide input. IMD 32 may respond to patient-supplied data from the user interface by altering therapy. For example, patient 14 may use external programmer 24 (e.g., a handheld device) to record (by pushing a button) a physiological event of interest. Processor circuitry 53 of IMD 32 may respond by turning the therapy on or off, or by adjusting the therapy (e.g., the stimulation strength) or by changing the therapy program. With reference to the urological applications discussed herein, patient 14 could push a button on external programmer 24 (e.g., their smartphone) when the bladder is voided. For example, this may send a signal to IMD 32 to turn off and enter hibernation mode for a period of time that is either pre-programmed based on the voiding characteristics of patient 14.

User interface 94 may include a button or keypad, lights, a speaker for voice commands, a display, such as a liquid crystal (LCD), light-emitting diode (LED), or cathode ray tube (CRT). In some examples the display may be a touch screen. As discussed in this disclosure, processor circuitry 90 may present and receive information relating to electrical stimulation and resulting therapeutic effects via user interface 94. For example, processor circuitry 90 may receive patient input via user interface 94. The input may be, for example, in the form of pressing a button on a keypad or selecting an icon from a touch screen.

Processor circuitry 90 may also present information to patient 14 in the form of alerts related to delivery of the electrical stimulation to patient 14 or a caregiver, as described in more detail below, via user interface 94. Although not shown, external programmer 24 may additionally or alternatively include a data or network interface to another computing device, to facilitate communication with the other device, and presentation of information relating to the electrical stimulation and therapeutic effects after termination of the electrical stimulation via the other device.

Telemetry circuitry 96 supports wireless communication between IMD 32 and external programmer 24 under the control of processor circuitry 90. Telemetry circuitry 96 may also be configured to communicate with another computing device via wireless communication techniques, or direct communication through a wired connection. In some examples, telemetry circuitry 96 may be substantially similar to telemetry circuitry 58 of IMD 32 described above, providing wireless communication via a radio frequency or proximal inductive medium. In some examples, telemetry circuitry 96 may include an antenna, which may take on a variety of forms, such as an internal or external antenna.

Examples of local wireless communication techniques that may be employed to facilitate communication between external programmer 24 and another computing device include RF communication according to the 802.11 or Bluetooth^{®} specification sets, infrared communication, e.g., according to the IrDA standard, or other standard or proprietary telemetry protocols. In this manner, other external devices may be capable of communicating with programmer 24 without needing to establish a secure wireless connection.

Power source 98 delivers operating power to the components of programmer 24. Power source 98 may include a battery and a power generation circuit to produce the operating power. In some examples, the battery may be rechargeable to allow extended operation.

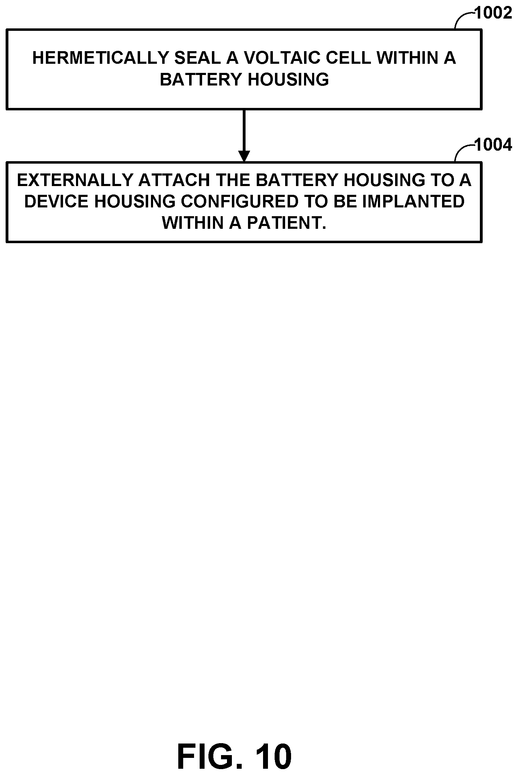

FIG. 10 is a flow diagram illustrating an example method of manufacturing a medical device. While described with respect to IMs 100-500, the techniques of FIG. 10 may be performed by IMDs 16 and/or 32 of FIGS. 7 and 8.

A manufacturer may hermetically seal a voltaic cell within a battery housing configured to be implantable within a patient (1002). For example, the manufacturer may hermetically seal a voltaic cell within battery housing 109 or battery housing 509 described above.

The manufacturer may externally attach the battery housing to a device housing configured to be implantable within a patient (1004). For example, the manufacturer may braze and/or weld battery housing 109 or 509 to device housing 102 or 302, respectively.

The techniques of this disclosure may be implemented in a wide variety of computing devices, medical devices, or any combination thereof. Any of the described units, circuitry or components may be implemented together or separately as discrete but interoperable logic devices. Depiction of different features as circuitry or units is intended to highlight different functional aspects and does not necessarily imply that such circuitry or units must be realized by separate hardware or software components. Rather, functionality associated with one or more circuitry or units may be performed by separate hardware or software components, or integrated within common or separate hardware or software components.

The disclosure contemplates computer-readable storage media comprising instructions to cause a processor to perform any of the functions and techniques described herein. The computer-readable storage media may take the example form of any volatile, non-volatile, magnetic, optical, or electrical media, such as a RAM, ROM, NVRAM, EEPROM, or flash memory that is tangible. The computer-readable storage media may be referred to as non-transitory. A server, client computing device, or any other computing device may also contain a more portable removable memory type to enable easy data transfer or offline data analysis.

The techniques described in this disclosure, including those attributed to various circuitry and various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated, discrete logic circuitry, or other processor circuitry, as well as any combinations of such components, remote servers, remote client devices, or other devices. The term "processor circuitry" or "processor circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. In addition, any of the described units, circuitry or components may be implemented together or separately as discrete but interoperable logic devices. Depiction of different features as circuitry or units is intended to highlight different functional aspects and does not necessarily imply that such circuitry or units must be realized by separate hardware or software components. Rather, functionality associated with one or more circuitry or units may be performed by separate hardware or software components, or integrated within common or separate hardware or software components. For example, any circuitry described herein may include electrical circuitry configured to perform the features attributed to that particular circuitry, such as fixed function processor circuitry, programmable processor circuitry, or combinations thereof.

The techniques described in this disclosure may also be embodied or encoded in an article of manufacture including a computer-readable storage medium encoded with instructions. Instructions embedded or encoded in an article of manufacture including a computer-readable storage medium encoded, may cause one or more programmable processors, or other processors, to implement one or more of the techniques described herein, such as when instructions included or encoded in the computer-readable storage medium are executed by the one or more processors. Example computer-readable storage media may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or any other computer readable storage devices or tangible computer readable media. The computer-readable storage medium may also be referred to as storage devices.

In some examples, a computer-readable storage medium comprises non-transitory medium. The term "non-transitory" may indicate that the storage medium is not embodied in a carrier wave or a propagated signal. In certain examples, a non-transitory storage medium may store data that may, over time, change (e.g., in RAM or cache).

This disclosure includes the following non-limiting examples.

Example 1. A medical device including: a device housing configured to be implantable within a patient, the device housing including an external surface in contact with a biocompatible electrical insulator: an electrical component housed within the device housing; and; and a battery external to the device housing and including a battery housing configured to be hermetically sealed, wherein the battery is configured to provide electrical power to the electrical component housed within the device housing, wherein the battery housing is configured to be attached to the device housing, wherein the battery housing includes: an internal surface in contact with a voltaic cell of the battery; and an external surface in contact with the biocompatible electrical insulator.an external surface in contact with the biocompatible electrical insulator.

Example 2: The medical device of example 1, wherein the internal surface and external surface are opposing surfaces of the same battery housing wall.

Example 3: The medical device of example 1 or example 2, wherein the battery housing includes a negative terminal of the battery.

Example 4: The medical device of example 3, further including an electrode positioned on an external surface of the device housing, wherein the electrode is configured to be in electrical contact with a tissue or a fluid of the patient, wherein the electrical component includes stimulation circuitry configured to deliver an electrical stimulation signal, and wherein the electrode is configured to be an electrical current source or an electrical current sink for the electrical stimulation signal.

Example 5: The medical device of any one of examples 1-4, wherein the battery housing is electrically isolated from the device housing by an electrical insulator.

Example 6: The medical device of any one of examples 1-5, wherein the battery housing is attached to the device housing by an electrical insulator.

Example 7: The medical device of example 5 or example 6, wherein the electrical insulator is a ceramic material.

Example 8: The medical device of any one of examples 1-7, wherein the device housing includes a ceramic material.

Example 9: The medical device of any one of examples 1-8, wherein the battery housing includes a ceramic material.

Example 10: The medical device of any one of examples 1-9, further including an electrode positioned on an external surface of the device housing, wherein the electrode is configured to be in electrical contact with a tissue or a fluid of the patient, and wherein the electrode is electrically isolated from the battery housing by the biocompatible electrical insulator.

Example 11: The medical device of any one of examples 1-10, wherein the biocompatible electrical insulator includes parylene.

Example 12: A medical device including: stimulation circuitry configured to generate an electrical stimulation signal; and a device housing configured to be implantable within a patient, wherein the device housing includes: a first portion housing the stimulation circuitry within a first volume; and a second portion housing a battery within a second volume, wherein the second portion forms a hermetic seal of the second volume, and wherein the battery is configured to provide electrical power to the stimulation circuitry.

Example 13: The medical device of example 12, wherein the second portion includes a negative terminal of the battery.

Example 14: The medical device of example 12 or example 13, further including an electrode positioned external to the device housing, wherein the electrode is configured to be in electrical contact with a tissue or a fluid of the patient and configured to be an electrical current source or an electrical current sink for the electrical stimulation signal.

Example 15: The medical device of any one of examples 12-14, wherein the second portion is electrically isolated from the first portion by an electrical insulator.

Example 16: The medical device of any one of examples 12-15, wherein the second portion is attached to the first portion by an electrical insulator, wherein the electrical insulator is a ceramic material.

Example 17: The medical device of any one of examples 12-16, wherein the first portion includes a ceramic material.

Example 18: The medical device of any one of examples 12-17, wherein the second portion includes a ceramic material.

Example 19: The medical device of example 14, wherein the electrode includes a leaded electrode, wherein the biocompatible electrical insulator includes parylene.

Example 20: A method of manufacturing a medical device including: hermetically sealing a voltaic cell within a battery housing configured to be implantable within a patient; and externally attaching the battery housing to a device housing configured to be implantable within a patient, wherein the battery housing includes: an internal surface in contact with a voltaic cell of the battery; and an external surface exposed to substantially the same environment as an external surface of the device housing.

Various examples have been described herein. Any combination of the described operations or functions is contemplated. These and other examples are within the scope of the following claims. Based upon the above discussion and illustrations, it is recognized that various modifications and changes may be made to the disclosed examples in a manner that does not require strictly adherence to the examples and applications illustrated and described herein. Such modifications do not depart from the true spirit and scope of various aspects of the disclosure, including aspects set forth in the claims.

## Claims

1. A medical device comprising:
a device housing configured to be implantable within a patient, the device housing comprising an external surface in contact with a biocompatible electrical insulator;
an electrical component housed within the device housing; and
a battery external to the device housing and comprising a battery housing configured to be hermetically sealed, wherein the battery is configured to provide electrical power to the electrical component housed within the device housing, wherein the battery housing is configured to be attached to the device housing, wherein the battery housing comprises:
an internal surface in contact with a voltaic cell of the battery; and
an external surface in contact with the biocompatible electrical insulator.

2. The medical device of claim 1, wherein the internal surface and external surface are opposing surfaces of the same battery housing wall.

3. The medical device of any of the preceding claims, wherein the battery housing comprises a negative terminal of the battery.

4. The medical device of any of the preceding claims, further comprising an electrode positioned on an external surface of the device housing, wherein the electrode is configured to be in electrical contact with a tissue or a fluid of the patient, wherein the electrical component comprises stimulation circuitry configured to deliver an electrical stimulation signal, and wherein the electrode is configured to be an electrical current source or an electrical current sink for the electrical stimulation signal.

5. The medical device of any of the preceding claims, wherein the battery housing is electrically isolated from the device housing by an electrical insulator and/or wherein the battery housing is attached to the device housing by an electrical insulator.

6. The medical device of claim 5, wherein the electrical insulator is a ceramic material.

7. The medical device of any of the preceding claims, wherein the device housing comprises a ceramic material and/or wherein the battery housing comprises a ceramic material.

8. The medical device of any of the preceding claims, further comprising an electrode positioned on an external surface of the device housing, wherein the electrode is configured to be in electrical contact with a tissue or a fluid of the patient, and wherein the electrode is electrically isolated from the battery housing by the biocompatible electrical insulator.

9. The medical device of any of the preceding claims, wherein the biocompatible electrical insulator comprises parylene.

10. A medical device comprising:
stimulation circuitry configured to generate an electrical stimulation signal; and
a device housing configured to be implantable within a patient,
wherein the device housing comprises:
a first portion housing the stimulation circuitry within a first volume; and
a second portion housing a battery within a second volume, wherein the second portion forms a hermetic seal of the second volume, and wherein the battery is configured to provide electrical power to the stimulation circuitry.

11. The medical device of claim 10, wherein the second portion comprises a negative terminal of the battery.

12. The medical device of any of the claims 10 or 11, further comprising an electrode positioned external to the device housing, wherein the electrode is configured to be in electrical contact with a tissue or a fluid of the patient and configured to be an electrical current source or an electrical current sink for the electrical stimulation signal.

13. The medical device of claim any of the claims 10 to 12, wherein the second portion is electrically isolated from the first portion by an electrical insulator, specifically, wherein the second portion is attached to the first portion by an electrical insulator.

14. The medical device of any of the claims 10 to 13, wherein the first portion comprises a ceramic material and/or wherein the second portion comprises a ceramic material.

15. A method of manufacturing a medical device, the method comprising:
hermetically sealing a voltaic cell within a battery housing configured to be implantable within a patient; and
externally attaching the battery housing to a device housing configured to be implantable within a patient,
wherein the battery housing comprises:
an internal surface in contact with a voltaic cell of the battery; and
an external surface exposed to substantially the same environment as an external surface of the device housing.
